# EUROPEAN PATENT APPLICATION

(11) **EP 3 766 868 A1**
(43) Date of publication of application: **20.01.2021**
(21) Application number: 19767617.4
(22) Date of filing: 13.03.2019
(51) Int. Cl.: C07D 209/88, B01J 31/22, C07B 61/00

(54) **METHOD FOR PRODUCING ANILINE DERIVATIVE**

(30) Priority: 16.03.2018 JP 2018049629
(71) Applicant: Nissan Chemical Corporation, Tokyo 103-6119 (JP)
(72) Inventor: TERAI Seiya, Funabashi-shi, Chiba 274-0052 (JP)
(74) Representative: Mewburn Ellis LLP
(86) International application number: PCT/JP2019/010353
(87) International publication number: WO 2019/177049

(57) **Abstract**

A method for producing a compound represented by formula (4) 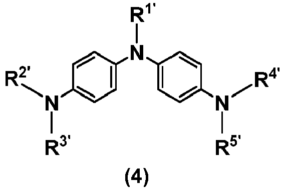 [R^{1'}-R^{5'} represent hydrogen atoms, formula (2), or formula (5), and at least one of R^{1'}-R^{5'} is a group represented by formula (5) (Ar¹-Ar³ are the same as below)], 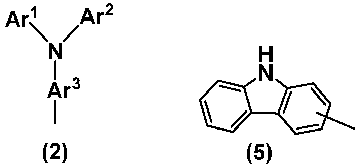 the method comprising carrying out deprotection after reacting an amine compound represented by formula (1) 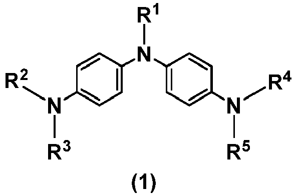 [R¹-R⁵ each independently represent a hydrogen atom or a group represented by formula (2) 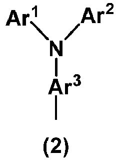 (Ar¹ and Ar² represent aryl groups and Ar³ represents an arylene group, and any two of Ar¹-Ar³ may bond to form a ring), and at least one of R¹-R⁵ is a hydrogen atom] with a compound represented by formula (3) 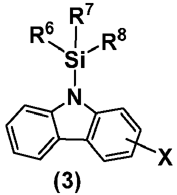 (X represents a halogen atom, etc., and R⁶-R⁸ represent an alkyl group, etc.) in the presence of a catalyst and a base, is a method for producing an aniline derivative suited to efficient industrial production that does not require a large excess of base and oxygen in the process of deprotection.

## Description

### TECHNICAL FIELD

The present invention relates to a method for producing an aniline derivative.

### BACKGROUND ART

Charge-transporting thin films consisting of organic compounds are used as light-emitting layers and charge-injecting layers in organic electroluminescent (hereinafter referred to as organic EL) devices. In particular, a hole-injecting layer is responsible for transferring charge between an anode and a hole-transporting layer or a light-emitting layer, and thus serves an important function in achieving low-voltage driving and high brightness in organic EL devices.

Methods for forming the hole-injecting layer are broadly divided into dry processes typified by vapor deposition and wet processes typified by spin coating, and comparing these different processes, wet processes are better able to efficiently produce thin films having a high flatness over a large area. Hence, with the progress currently underway toward larger-area organic EL displays, there exists a desire for a hole-injecting layer that can be formed by a wet process.

In view of these circumstances, the inventors have developed aniline derivatives which can be employed in various wet processes and which moreover give thin films that, when used as hole-injecting layers for organic EL devices, are capable of achieving excellent EL device characteristics, and aniline derivatives which have a good solubility in organic solvents used in such aniline derivatives (see, for example, Patent Document 1).

Patent Document 1 discloses, as one of the methods for producing such an aniline derivative, for example, as shown in the following scheme, a method of coupling amine compound (A) with halogenated carbazole compound (B) in which an NH group is protected with a benzyl group in the presence of a catalyst, and then deprotecting the benzyl group.

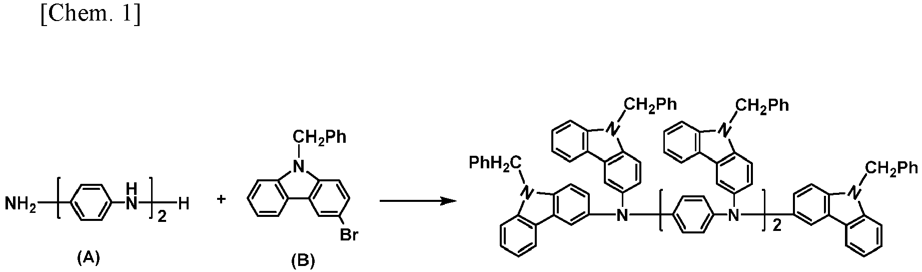

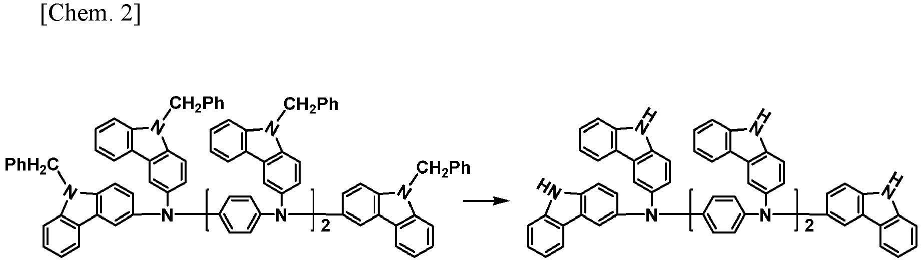

However, when the carbazole compound protected with a benzyl group is used, there has been a problem that a large excess of base and oxygen is required in the deprotection step, which is not suitable as an industrial production method.

### PRIOR ART DOCUMENTS

### PATENT DOCUMENTS

Patent Document 1: WO 2015/050253

### SUMMARY OF INVENTION

### TECHNICAL PROBLEM

The invention has been made in view of the above circumstances, and an object of the invention is to provide a method for producing an aniline derivative suitable for efficient and industrial methods, that does not require a large excess of base and oxygen in the process of deprotection.

### SOLUTION TO PROBLEM

The inventors have conducted extensive investigations in order to achieve the above object, as a result of which they have found that an aniline derivative can be efficiently produced without requiring a large excess of base and oxygen by causing a coupling reaction of an amine compound with a carbazole compound in which an NH group is protected with a predetermined silyl group, followed by desilylation, thereby completing the invention.

That is, the invention provides:
1. a method for producing an aniline derivative of formula (4): 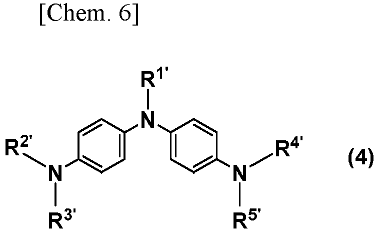 wherein R^{1'} to R^{5'} are each independently a hydrogen atom, a group of formula (2), or a group of formula (5), and at least one of R^{1'} to R^{5'} is a group of formula (5): 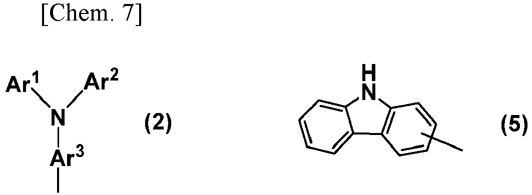 (wherein Ar¹ to Ar³ have the same meaning as below)
   the method including causing a coupling reaction of an amine compound of formula (1): 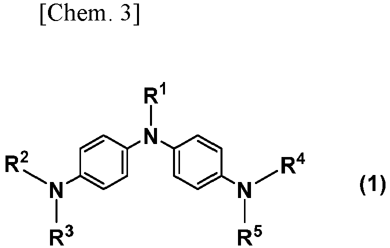 wherein R¹ to R⁵ are each independently a hydrogen atom or a group of formula (2): 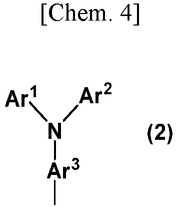 (wherein Ar¹ and Ar² are each independently an aryl group of 6 to 20 carbon atoms, Ar³ is an arylene group of 6 to 20 carbon atoms, and any two of Ar¹ to Ar³ may be bonded to form a ring with a nitrogen atom)
   and at least one of R¹ to R⁵ is a hydrogen atom;
   with a carbazole compound of formula (3): 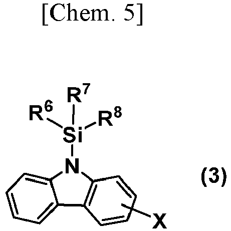 wherein X is a halogen atom or a pseudohalogen group, R⁶ to R⁸ are alkyl groups of 1 to 20 carbon atoms or aryl groups of 6 to 20 carbon atoms which may be substituted with Z¹, and Z¹ is an alkoxy group of 1 to 20 carbon atoms, a halogen atom, a nitro group, or a cyano group,
   in the presence of a catalyst and a base, followed by deprotecting the silyl group;
2. the method for producing an aniline derivative of 1, wherein R⁶ to R⁸ are alkyl groups of 1 to 10 carbon atoms or aryl groups of 6 to 10 carbon atoms;
3. the method for producing an aniline derivative of 2, wherein two of R⁶ to R⁸ are methyl groups and the remaining one is a t-butyl group;
4. the method for producing an aniline derivative of any one of 1 to 3, wherein the base is sodium t-butoxide;
5. the method for producing an aniline derivative of any one of 1 to 4, wherein the catalyst is a palladium catalyst;
6. the method for producing an aniline derivative of any one of 1 to 5, wherein the coupling reaction is performed at 40 to 100°C;
7. the method for producing an aniline derivative of any one of 1 to 6, wherein the deprotection is performed using a fluoride ion; and
8. the method for producing an aniline derivative of any one of 1 to 7, wherein R¹ to R⁵ are all hydrogen atoms.

### ADVANTAGEOUS EFFECTS OF INVENTION

In the method for producing an aniline derivative of the invention, since a raw material in which an NH group of a carbazole compound is protected with a silyl group is used, a large excess of base and oxygen is not required in the process of deprotection, and a target aniline derivative can be efficiently produced.

### BRIEF DESCRIPTION OF DRAWINGS

[FIG. 1] FIG. 1 is a HPLC chart of CZ5 obtained in Example 1.
[FIG. 2] FIG. 2 is an enlarged view of the range of 0 to 300 mAU on the vertical axis in FIG. 1.
[FIG. 3] FIG. 3 is a HPLC chart of CZ5 obtained in Comparative Example 1.
[FIG. 4] FIG. 4 is an enlarged view of the range of 0 to 300 mAU on the vertical axis in FIG. 3.

### DESCRIPTION OF EMBODIMENTS

Hereinafter, the invention is described in more detail.

The method for producing an aniline derivative according to the invention includes causing a coupling reaction of an amine compound of formula (1) with a carbazole compound of formula (3) in the presence of a catalyst and a base, followed by deprotecting a silyl group on a nitrogen atom of a carbazole moiety.

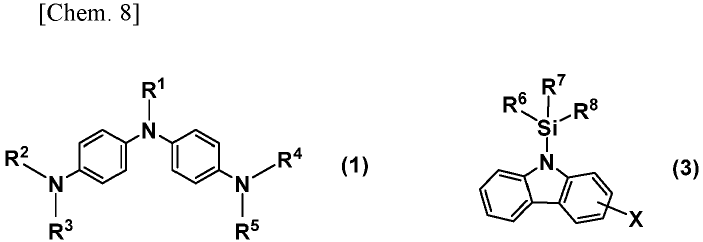

In the above formula (1), R¹ to R⁵ each independently represent a hydrogen atom or a group of formula (2), and in order to cause a coupling reaction with the carbazole compound of formula (3), at least one of R¹ to R⁵ is required to be a hydrogen atom.

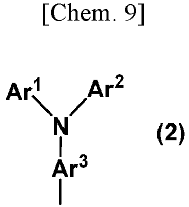

In formula (2), Ar¹ and Ar² are each independently an aryl group of 6 to 20 carbon atoms, Ar³ is an arylene group of 6 to 20 carbon atoms, and any two of Ar¹ to Ar³ may be bonded to form a ring with a nitrogen atom.

Specific examples of the aryl group of 6 to 20 carbon atoms include phenyl, 1-naphthyl, 2-naphthyl, 1-anthryl, 2-anthryl, 9-anthryl, 1-phenanthryl, 2-phenanthryl, 3-phenanthryl, 4 phenanthryl, and 9-phenanthryl groups.

Specific examples of the arylene group of 6 to 20 carbon atoms include benzene-1,2-diyl(o-phenylene), benzene-1,3-diyl(m-phenylene), benzene-1,4-diyl(p-phenylene), naphthalene-1,2-diyl, naphthalene-1,3-diyl, naphthalene-1,4-diyl, naphthalene-1,5-diyl, naphthalene-1,6-diyl, naphthalene-1,7-diyl, and naphthalene-1,8-diyl groups.

In addition, the ring formed by combining any two of Ar¹ to Ar³ with a nitrogen atom include a carbazole ring and the like.

Of these, Ar¹ and Ar² are preferably a phenyl group, a 1-naphthyl group, and a 2-naphthyl group, and more preferably a phenyl group.

Further, Ar³ is preferably a benzene-1,2-diyl group, a benzene-1,3-diyl group, or a benzene-1,4-diyl group, and more preferably a benzene-1,4-diyl group.

Therefore, the group of formula (2) is preferably a group of formula (2A), and more preferably a group of formula (2A-1).

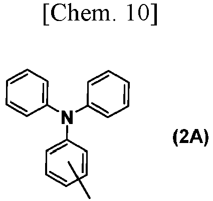

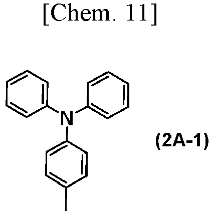

Examples of the compound of formula (1) which can be preferably used in the production method of the invention include a compound of the following formula (1A) in which R¹ to R⁵ are all hydrogen atoms, compounds of the following formula (1B) in which R² and R⁴ are 4-diphenylaminophenyl groups, and compounds of the following formula (1C) in which R¹, R², and R⁴ are 4-diphenylaminophenyl groups, but are not limited thereto.

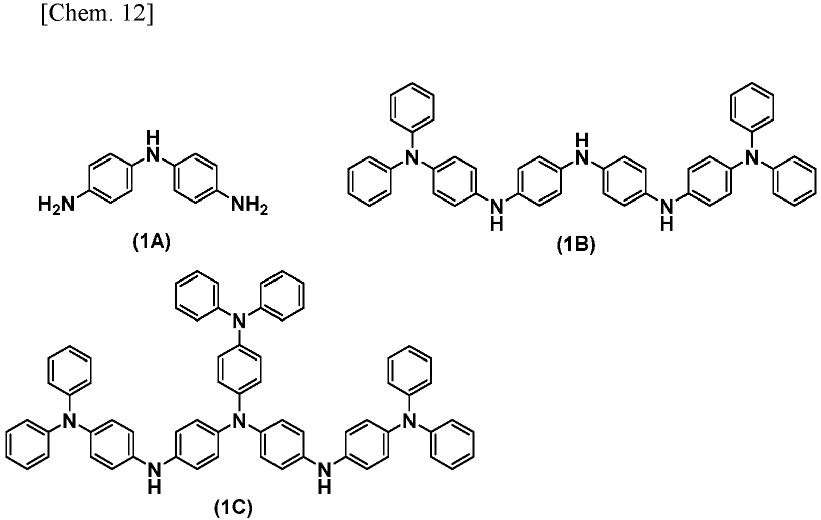

On the other hand, X in formula (3) is a halogen atom or a pseudohalogen group, and R⁶ to R⁸ are alkyl groups of 1 to 20 carbon atoms or aryl groups of 6 to 20 carbon atoms which may be substituted with Z¹, and Z¹ is an alkoxy group of 1 to 20 carbon atoms, a halogen atom, a nitro group, or a cyano group.

Examples of the halogen atom include fluorine, chlorine, bromine, and iodine atoms.

Examples of the pseudohalogen group include (fluoro)alkylsulfonyloxy groups such as methanesulfonyloxy, trifluoromethanesulfonyloxy, and nonafluorobutanesulfonyloxy groups; and aromatic sulfonyloxy groups such as benzenesulfonyloxy and toluenesulfonyloxy groups.

Of these, X is preferably a halogen atom, and more preferably a bromine atom or an iodine atom, in consideration of availability and reactivity of raw materials and the like.

Also, the substitution position of X is not particularly limited, but is preferably para to the nitrogen atom of carbazole.

The alkyl group of 1 to 20 carbon atoms may be linear, branched, or cyclic, and examples include linear or branched alkyl groups of 1 to 20 carbon atoms, such as methyl, ethyl, n-propyl, isopropyl, n-butyl, isobutyl, s-butyl, t-butyl, n-pentyl, n-hexyl, n-heptyl, n-octyl, n-nonyl, and n-decyl groups; and cyclic alkyl groups of 3 to 20 carbon atoms, such as cyclopropyl, cyclobutyl, cyclopentyl, cyclohexyl, cycloheptyl, cyclooctyl, cyclononyl, cyclodecyl, bicyclobutyl, bicyclopentyl, bicyclohexyl, bicycloheptyl, bicyclooctyl, bicyclononyl, and bicyclodecyl groups.

Examples of the aryl group having 6 to 20 carbon atoms include similar ones to the groups described above.

For the alkoxy group of 1 to 20 carbon atoms, the alkyl group therein may be linear, branched, or cyclic, and examples include linear or branched alkoxy groups of 1 to 20 carbon atoms, such as methoxy, ethoxy, n-propoxy, isopropoxy, n-butoxy, t-butoxy, n-pentyloxy, n-hexyloxy, n-heptyloxy, n-octyloxy, n-nonyloxy, and n-decyloxy groups; and cyclic alkyloxy group of 3 to 20 carbon atoms, such as cyclopentyloxy and cyclohexyloxy groups.

Of these, R⁶ to R⁸ are preferably alkyl groups of 1 to 10 carbon atoms or aryl groups of 6 to 10 carbon atoms, and more preferably alkyl groups of 1 to 5 carbon atoms.

More specifically, a combination of two of R⁶ to R⁸ being methyl groups and the remaining one being a t-butyl group, a combination of two of R⁶ to R⁸ being phenyl groups and the remaining one being a t-butyl group, and all of R⁶ to R⁸ being isopropyl groups are preferred, and a combination of two of R⁶ to R⁸ being methyl groups and the remaining one being a t-butyl group is more preferred.

Examples of the compound of formula (3) which can be preferably used in the production method of the invention include a compound of the following formula (3A), particularly a compound of formula (3A-1), but are not limited thereto.

The compound of formula (3) can be obtained by a known method of reacting the corresponding carbazole with a triorganohalide such as trialkylsilyl chloride in the presence of a base such as NaH. 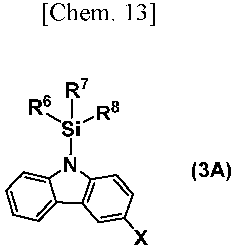 wherein R⁶ to R⁸ and X have the same meanings as above.

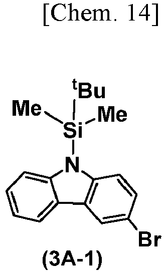

In the coupling reaction between the amine compound of the above formula (1) and the carbazole compound of formula (3), the charging ratio of the amine compound of formula (1) and the carbazole compound of formula (3) is, in the amount of substance (mol) ratio, preferably about 1 to 5 carbazole compounds, and more preferably about 1.1 to 2, with respect to 1 target NH group to be reacted with the amine compound.

Examples of the catalyst used in the reaction include copper catalysts such as copper chloride, copper bromide, and copper iodide; and palladium catalysts such as Pd(PPh₃)₄ (tetrakis(triphenylphosphine)palladium), Pd(PPh₃)₂Cl₂ (bis(triphenylphosphine)dichloropalladium), Pd(dba)₂ (bis(benzylideneacetone)palladium), Pd₂(dba)₃ (tris(benzylideneacetone)dipalladium), Pd(P-t-Bu₃)₂ (bis(tri(t-butylphosphine))palladium), and Pd(OAc)₂ (palladium acetate). These catalysts may be used singly, or two or more may be used in combination.

Also, these catalysts may be used together with suitable known ligands.

Examples of such ligands include tertiary phosphines such as triphenylphosphine, tri-o-tolylphosphine, diphenylmethylphosphine, phenyldimethylphosphine, trimethylphosphine, triethylphosphine, tributylphosphine, tri-t-butylphosphine, di-t-butyl(phenyl)phosphine, di-t-butyl(4-dimethylaminophenyl)phosphine, 1,2-bis(diphenylphosphino)ethane, 1,3-bis(diphenylphosphino)propane, 1,4-bis(diphenylphosphino)butane, and 1,1'-bis(diphenylphosphino)ferrocene; and tertiary phosphites such as trimethylphosphite, triethylphosphite, and triphenylphosphite, and di-t-butyl(phenyl)phosphine is preferably used in the invention.

The amount of catalyst used can be each set to about 0.1 to 100 mol% per mole of the target NH group to be reacted with the amine compound of formula (1), and is preferably about 1 to 10 mol%, more preferably about 2 to 5 mol%, and even more preferably about 2 mol%.

In addition, when ligands are used, the amount thereof can be set to 0.1 to 5 equivalents, and is preferably 1 to 2 equivalents, per the metal complex used.

Further, examples of the base include alkali metal simple substances such as lithium, sodium, potassium, lithium hydride, sodium hydride, potassium hydroxide, t-butoxy lithium, t-butoxy sodium, t-butoxy potassium, lithium hydroxide, sodium hydroxide, potassium hydroxide, sodium carbonate, potassium carbonate, sodium hydrogen carbonate, and potassium hydrogen carbonate, alkali metal hydroxides, alkoxy alkali metals, alkali metal carbonates, alkali metal hydrogen carbonates; and alkaline earth metal carbonates such as calcium carbonate, and in consideration of efficient progress of the coupling reaction, t-butoxy sodium is preferred.

The amount of base used is preferably about 1 to 2 equivalents, and more preferably about 1.2 to 1.5 equivalents, per the target NH group to be reacted with the amine compound of formula (1).

In cases where starting compounds are all solids or from the standpoint of efficiently obtaining the target coupling product, each of the above reactions is carried out in a solvent. When a solvent is used, the type thereof is not particularly limited, provided that it does not have an adverse influence on the reaction. Specific examples thereof include aliphatic hydrocarbons (pentane, n-hexane, n-octane, n-decane, decalin, etc.), halogenated aliphatic hydrocarbons (chloroform, dichloromethane, dichloroethane, carbon tetrachloride, etc.), aromatic hydrocarbons (benzene, nitrobenzene, toluene, o-xylene, m-xylene, p-xylene, mesitylene, etc.), halogenated aromatic hydrocarbons (chlorobenzene, bromobenzene, o-dichlorobenzene, m-dichlorobenzene, p-dichlorobenzene, etc.), ethers (diethyl ether, diisopropyl ether, t-butyl methyl ether, tetrahydrofuran, dioxane, 1,2-dimethoxyethane, 1,2-diethoxyethane, etc.), ketones (acetone, methyl ethyl ketone, methyl isobutyl ketone, di-n-butyl ketone, cyclohexanone, etc.), amides (N,N-dimethylformamide, N,N-dimethylacetamide, etc.), lactams and lactones (N-methylpyrrolidone, γ-butyrolactone, etc.), urea (N,N-dimethylimidazolidinone, tetramethylurea, etc.), sulfoxides (dimethylsulfoxide, sulfolane, etc.), and nitriles (acetonitrile, propionitrile, butyronitrile, etc.). These solvents may be used singly, or two or more may be used in admixture.

Of these, aromatic hydrocarbons are particularly preferred, and toluene is more preferred.

The reaction temperature may be suitably set in the range of the melting point to the boiling point of the solvent to be used, and is preferably about 0 to 200°C, and more preferably about 20 to 150°C, and in consideration of enhancing the yield of the coupling product, even more preferably about 40 to 100°C.

After completion of the reaction, the coupling product can be obtained by post-treatment in the usual manner.

Subsequently, the silyl group on the nitrogen atom of the carbazole moiety in the obtained coupling product is deprotected.

Deprotection can be performed by appropriately selecting from known methods, but in the invention, deprotection with fluoride ions is preferred.

A desilylation agent that serves as a fluoride ion source is not particularly limited and can be appropriately selected from known ones and used.

Specific examples thereof include tetrabutylammonium fluoride (TBAF), tetraethylammonium fluoride, tetramethylammonium fluoride and hydrates thereof, hydrogen fluoride pyridine complex, cesium fluoride, potassium fluoride, potassium hydrogen fluoride, sodium fluoride, lithium fluoride, and calcium fluoride, and of these, TBAF is preferred.

The amount of desilylation agent used can be set to about 1 to 5 equivalents, and is preferably about 1.2 to 2 equivalents, per the total N-Si bonds in the coupling product.

A solvent can be used also in this reaction, and specific examples of the usable solvent are as described above, but ethers are preferred, and tetrahydrofuran is more preferred.

The reaction temperature can range from the melting point to the boiling point of the solvent, but is preferably about 0 to 100°C, more preferably about 0 to 30°C, and even more preferably at a room temperature of about 25°C.

After completion of the reaction, the target aniline derivative of the following formula (4) can be obtained by post-treatment in the usual manner.

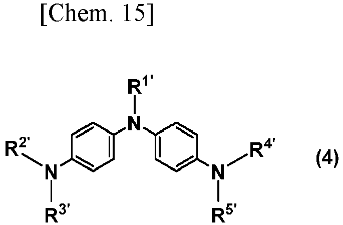

In the formula, R^{1'} to R^{5'} are each independently a hydrogen atom, a group of the above formula (2), or a group of the following formula (5), and at least one of R^{1'} to R^{5'} is a group of formula (5).

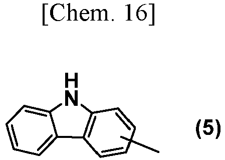

Examples of suitable aniline derivatives obtained by the production method of the invention include those of the following formulas (4A) to (4D), but are not limited thereto.

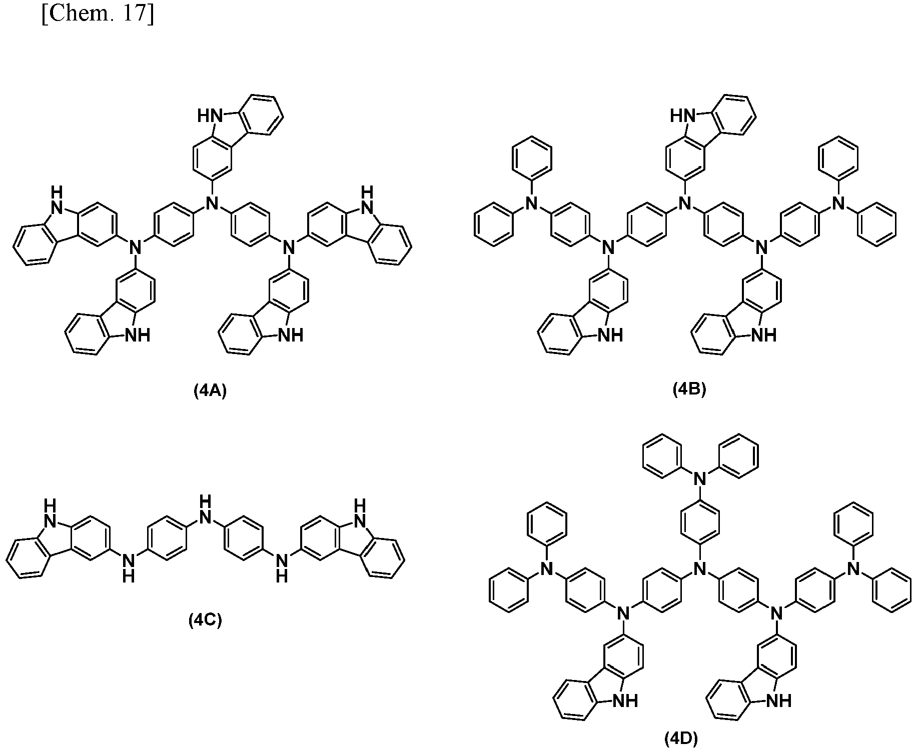

### EXAMPLES

Hereinafter, the present invention is described more specifically with reference to Examples, but the present invention is not limited to the following Examples.

Here, ¹H-NMR was measured using a nuclear magnetic resonance spectrometer AVANCE III HD 500 MHz manufactured by Bruker BioSpin Corporation.

High performance liquid chromatography (HPLC) was measured under the following measurement conditions using Prominence manufactured by Shimadzu Corporation.
Eluent A: 0.05 volume% Aqueous trifluoroacetic acid solution
Eluent B: Acetonitrile/tetrahydrofuran
   (1:1 volume conversion, 0.05 volume% trifluoroacetic acid added)
Flow rate: 0.5 mL/min
Column: Poroshell 120 EC-C8 (2.7 µm, 3.0 × 50 mm, Agilent Technologies, Inc.
Gradation conditions:
   Eluent B: 30% (0 to 0.01 min)
   → 30 to 100% (0.01 to 10 min) → 100% (10 to 30 min)

### [Example 1] Synthesis of aniline derivative Cz5

### (1) Synthesis of TBSCZ5

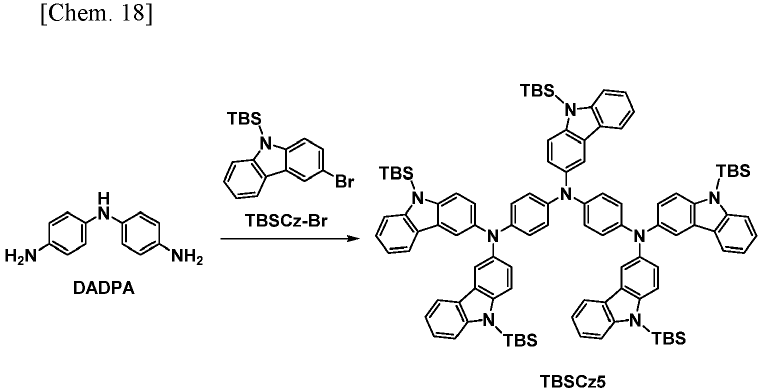

A flask was charged with 4.78 g of DADPA, 47.6 g of TBSCZ-Br, 1.38 g of Pd(dba)₂, and 16.6 g of sodium t-butoxide, following which the interior of the flask was replaced with nitrogen. Next, 240 mL of toluene and 20 mL of a previously prepared toluene solution of di-t-butyl(phenyl)phosphine (concentration: 55 g/L) were added thereto, and the mixture was stirred at 90°C. After 2.5 hours, the reaction solution was cooled to room temperature, 240 mL of ion-exchanged water was mixed, and liquid separation treatment was carried out. The organic layer was further separated and washed with ion-exchanged water and saturated saline, then dried over sodium sulfate, and concentrated. The resulting crude product was dissolved in 200 mL of toluene, and 3.5 g of Shirasagi P activated carbon (manufactured by Osaka Gas Chemicals Co., Ltd.) was added thereto. The mixture was stirred at room temperature for 2 hours and then filtered through silica gel, and cake washing was carried out with toluene. After the filtrate was concentrated to 240 g, the concentrate was added dropwise to a premixed solution of 7.02 g of N,N-diethyldithiocarbamate sodium trihydrate, 1.20 L of methanol, and 0.40 L of ethyl acetate, and the resulting slurry was stirred at room temperature. After 18 hours, the slurry was filtered, and the filter product was washed with methanol and then dried to obtain TBSCZ5 (yield: 36.3 g, yield: 95%). The measurement result of ¹H-NMR is shown below.
¹H-NMR (500 MHz, THF-d₈) δ[ppm]:
7.88-7.98 (m, 10H), 7.55-7.61 (m, 10H), 7.19-7.30 (m, 10H), 7.12 (t, J = 7.3 Hz, 1H), 7.05 (t, J = 7.3 Hz, 4H), 6.96-7.00 (m, 8H), 1.05 (s, 36H), 1.04 (s, 9H), 0.74 (s, 30H).

Here, TBSCZ-Br of the following formula was synthesized according to Chemistry of Materials (2015), 27(19), 6535-6542. (hereinafter the same).

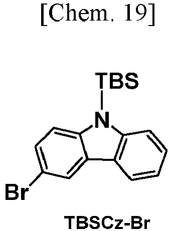

### (2) Synthesis of CZ5

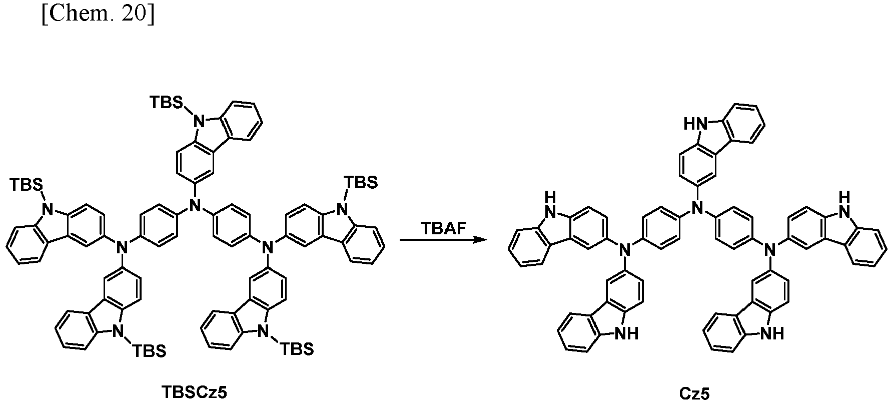

A flask was charged with 178 mL of THF and 33.5 g of TBSCZ5, then the mixture was stirred while cooling with an ice bath, and 158 mL of a THF solution of TBAF (concentration: 1 M) was added dropwise. After completing the dropwise addition, the ice bath was removed, and the mixture was stirred at room temperature for 1.5 hours, then the reaction solution was added dropwise to 670 mL of ion-exchanged water to quench. The resulting precipitate was filtered and then converted into 130 g of a THF solution, and the solution was added dropwise to 1 L of methanol to precipitate again. The operation from filtration to crystallization was repeated twice more, and the precipitate was collected by filtration and dried to obtain CZ5 (yield: 18.6 g, yield: 87%).

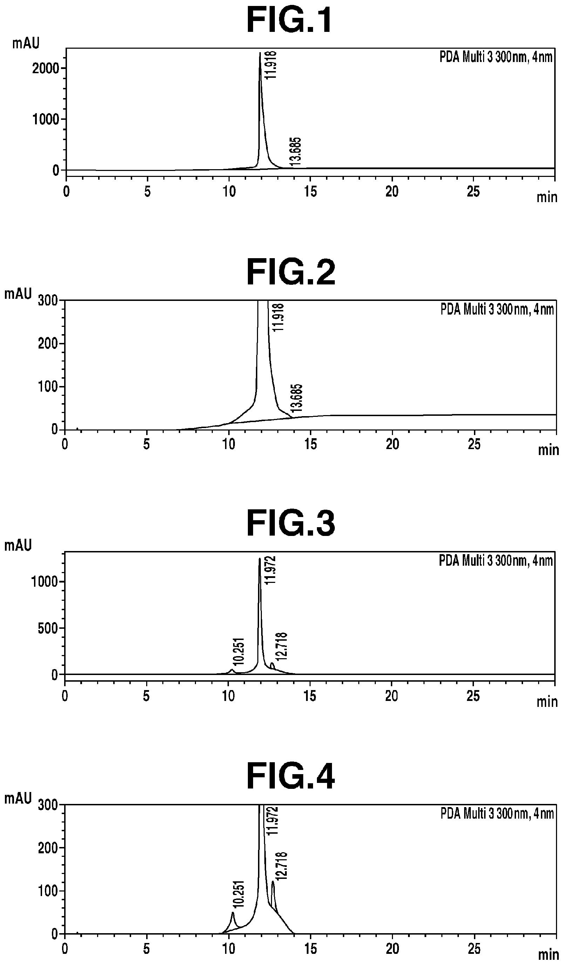

The HPLC chart of the obtained CZ5 is shown in FIGS. 1 and 2.

### [Example 2] Synthesis of aniline derivative 3Cz-TRI3

### (1) Synthesis of 3TBSCz-TRI3

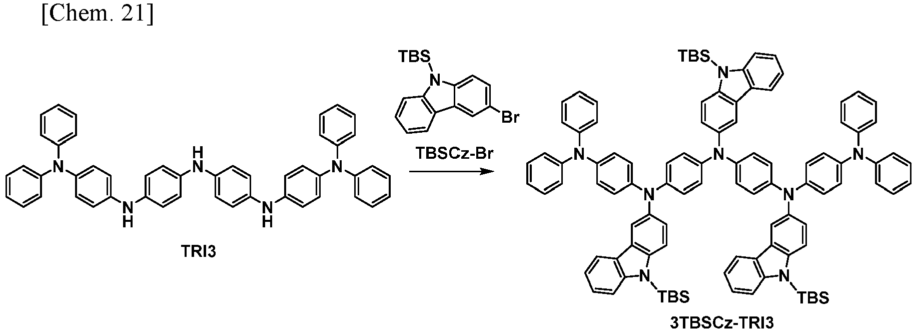

A flask was charged with 55.1 g of TRI3, 95.4 g of TBSCZ-Br, 2.77 g of Pd(dba)₂, and 32.4 g of sodium t-butoxide, following which the interior of the flask was replaced with nitrogen. Next, 550 mL of toluene and 22 mL of a previously prepared toluene solution of di-t-butyl(phenyl)phosphine (concentration: 96 g/L) were added thereto, and the mixture was stirred at 90°C. The reaction solution was cooled to room temperature for 1.5 hours, 550 mL of ion-exchanged water was mixed, and liquid separation treatment was carried out. To the resulting organic layer was added 11.0 g of Shirasagi P activated carbon (manufactured by Osaka Gas Chemicals Co., Ltd.), and the mixture was stirred at room temperature for 1 hour and then filtered through silica gel, and cake washing was carried out with toluene. The filtrate was concentrated to 660 g, the concentrate was added dropwise to a premixed solution of 22.0 g of N,N-diethyldithiocarbamate sodium trihydrate, 3.30 L of methanol, and 1.10 L of ethyl acetate, and the resulting slurry was stirred at room temperature. After 1 hour, the slurry was filtered, and the filter product was washed with methanol and then dried to obtain 3TBSCZ-TRI3 (yield: 112 g, yield: 92%). The measurement result of ¹H-NMR is shown below.
¹H-NMR (500 MHz, THF-d₈) δ[ppm]:
7.93-7.95 (m, 3H), 7.89 (d, J = 2.1 Hz, 3H), 7.57-7.62 (m, 6H), 7.26-7.30 (m, 3H), 7.15-7.21 (m, 11H), 7.06-7.13 (m,3H), 6.96-7.03 (m, 20H), 6.88-6.94 (m, 8H), 1.05 (s, 18H), 1.04 (s, 9H), 0.75 (s, 12H), 0.74 (s, 6H).

### (3) Synthesis of 3Cz-TRI3

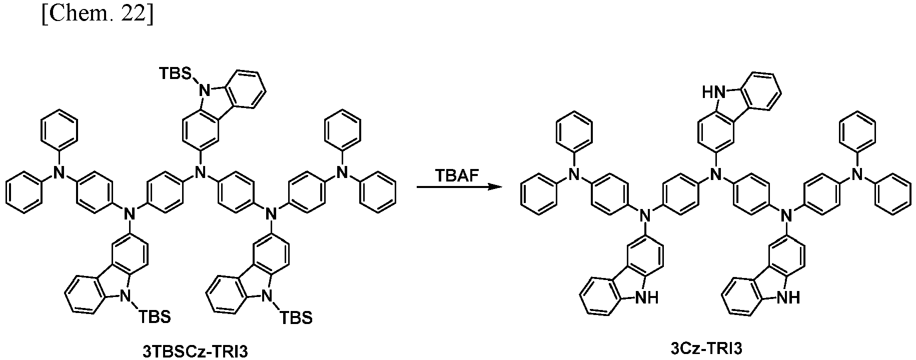

A flask was charged with 315 mL of THF and 105 g of 3TBSCZ-TRI3, then the mixture was stirred while cooling with an ice bath, and 310 mL of a THF solution of TBAF (concentration: 1 M) was added dropwise. After completing the dropwise addition, the ice bath was removed, and the mixture was stirred at room temperature for 3 hours. Thereafter, the reaction solution was diluted with THF to 735 g, and this was added dropwise to 1.57 L of methanol to quench. The resulting precipitate was stirred at room temperature for 1 hour and then filtered, and cake washing was carried out with methanol. Thereafter, the resulting mixture was further subjected to slurry washing with 1.05 L of methanol for 2 hours. This was collected by filtration, and cake washing was carried out with methanol and then dried to obtain 3CZ-TRI3 (yield: 75.4 g, yield: 93%).

### [Example 3] Synthesis of aniline derivative 3TPA-CZ3

### (1) Synthesis of TBSCZ3

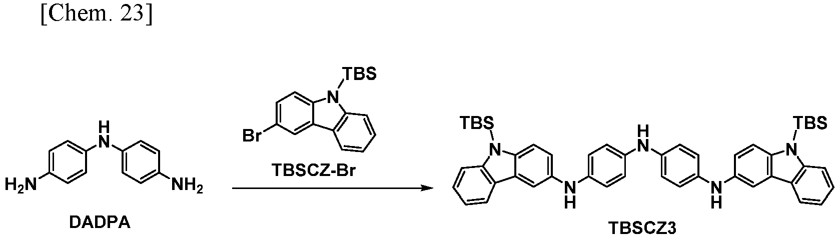

A flask was charged with 1.99 g of DADPA, 7.22 g of TBSCZ-Br, 233 mg of Pd(dba)₂, and 2.69 g of sodium t-butoxide , following which the interior of the flask was replaced with nitrogen. Next, 100 mL of toluene and 3.7 mL of a previously prepared toluene solution of di-t-butyl(phenyl)phosphine (concentration: 49 g/L) were added thereto, and the mixture was stirred at 90°C. After 2 hours, the reaction solution was cooled to room temperature, mixed with 100 mL of ion-exchanged water, and then filtered. The resulting filter product was dissolved in hot toluene, filtered while hot, and the filtrate was stirred at room temperature. After 18 hours, the precipitate was collected by filtration, washed with methanol, and then dried to obtain TBSCZ3 (yield: 3.87 g, yield: 51%). The measurement result of ¹H-NMR is shown below.
¹H-NMR (500 MHz, DMSO-d₆) δ[ppm]:
7.99 (d, J = 7.6 Hz, 2H), 7.68 (d, J = 2.1 Hz, 2H), 7.63 (s, 2H), 7.59 (d, J = 8.5 Hz, 2H), 7.52 (d, J = 8.9 Hz, 2H), 7.49 (s, 1H), 7.30-7.33 (m, 2H), 7.12-7.15 (m, 2H), 7.07 (dd, J = 8.9, 2.1 Hz, 2H), 6.94-7.02 (m, 8H), 0.98 (s, 18H), 0.73 (s, 12H).

### (2) Synthesis of 3TPA-TBSCZ3

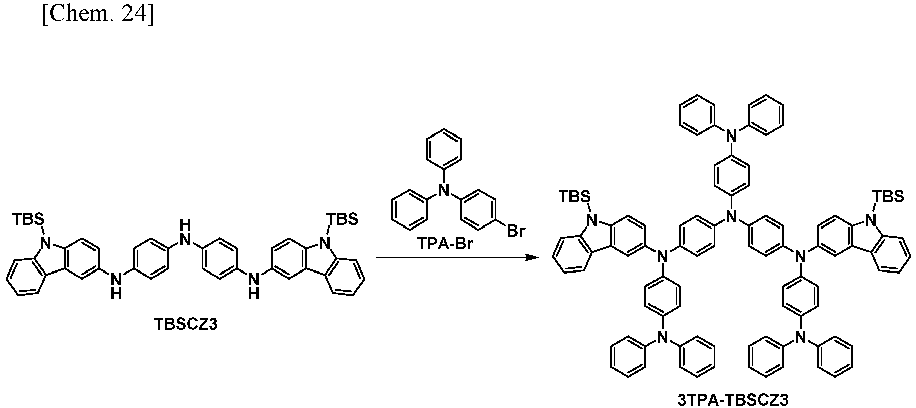

A flask was charged with 2.65 g of TBSCZ3, 3.74 g of TPA-Br, 121 mg of Pd(dba)₂, and 1.42 g of sodium t-butoxide, following which the interior of the flask was replaced with nitrogen. Next, 80 mL of toluene and 1.6 mL of a previously prepared toluene solution of di-t-butyl(phenyl)phosphine (concentration: 60 g/L) were added thereto, and the mixture was stirred at 90°C. After 4 hours, the reaction solution was cooled to room temperature, 100 mL of ion-exchanged water was mixed, and liquid separation treatment was carried out. Further, the organic layer was washed with an aqueous solution of 5% sodium N,N-diethyldithiocarbamate trihydrate, ion-exchanged water, and saturated saline, and then 0.1 g of Shirasagi P activated carbon (manufactured by Osaka Gas Chemicals Co., Ltd.) was added thereto. The mixture was stirred at room temperature for 1 hour and then filtered through silica gel, and cake washing was carried out with toluene. After the filtrate was concentrated, the resulting crude product was dissolved in 50 mL of toluene and added dropwise to a premixed solution of 375 mL of methanol and 125 mL of ethyl acetate, and the resulting slurry was stirred at room temperature. After 3 days, the slurry was filtered, and the filter product was dried to obtain 3TPA-TBSCZ3 (yield: 4.80 g, yield: 92%). The measurement result of ¹H-NMR is shown below.
¹H-NMR (500 MHz, THF-d₈) δ[ppm]:
7.94 (d, J = 7.3 Hz, 2H), 7.89 (d, J = 1.8 Hz, 2H), 7.61 (t, J = 8.2 Hz, 4H), 7.29 (t, J = 7.3 Hz, 2H), 7.16-7.20 (m, 14H), 7.10 (t, J = 7.3 Hz, 2H), 6.98-7.04 (m, 26H), 6.89-6.94 (m, 12H).

### (3) Synthesis of 3TPA-CZ3

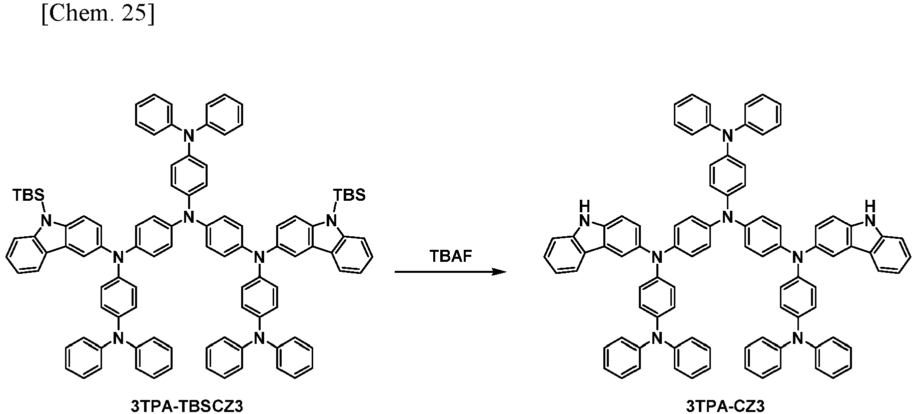

A flask was charged with 22.5 mL of THF and 3.72 g of 3TPA-TBSCZ3, and then 7.5 mL of a THF solution of TBAF (concentration: 1 M) was added dropwise with stirring. One hour after the completion of the dropwise addition, the reaction solution was added dropwise to 300 mL of methanol to quench, and the resulting precipitate was collected by filtration. This was dissolved by heating in a mixed solution of 90 mL of toluene and 10 mL of 1,4-dioxane, filtered while hot, and then recrystallized. The crystals were further subjected to slurry washing with 50 mL of methanol, and collected by filtration, then dried to obtain 3TPA-CZ3 (yield: 1.78 g, yield: 57%). The measurement result of ¹H-NMR is shown below.
¹H-NMR (500 MHz, DMSO-d₆) δ[ppm]:
11.26 (s,2H), 8.03 (d, J = 7.6 Hz, 2H), 7.95 (d, J = 1.5 Hz, 2H), 7.47 (t, J = 8.9 Hz, 4H), 7.36 (t, J = 7.6 Hz, 2H), 7.17-7.26 (m, 14H), 7.08 (t, J = 7.6 Hz, 2H), 6.88-6.97 (m, 38H).

### [Comparative Example 1]

Cz5 was synthesized according to WO 2015/050253 (Patent Document 1). The HPLC chart of the obtained CZ5 is shown in FIGS. 3 and 4.

As shown in FIGS. 1 to 4, comparing Cz5 synthesized in Example 1 (FIGS. 1 and 2) with Cz5 synthesized in Comparative Example 1 (FIGS. 3 and 4), the HPLC area percentage of Cz5 synthesized in Example 1 was 99.9%, whereas that of Cz5 synthesized in Comparative Example 1 was 93.5%.

From the difference in the HPLC area percentage of the obtained Cz5, it was found that the invention provides an efficient method for producing an aniline derivative suitable for mass production and simultaneously provides a highly pure compound.

## Claims

1. A method for producing an aniline derivative of formula (4): 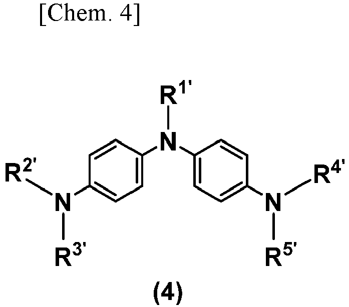 wherein R^{1'} to R^{5'} are each independently a hydrogen atom, a group of formula (2), or a group of formula (5), and at least one of R^{1'} to R^{5'} is a group of formula (5): 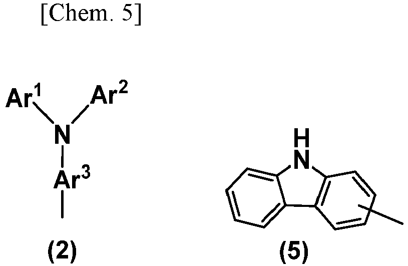 (wherein Ar¹ to Ar³ have the same meaning as below)
the method comprising causing a coupling reaction of an amine compound of formula (1): 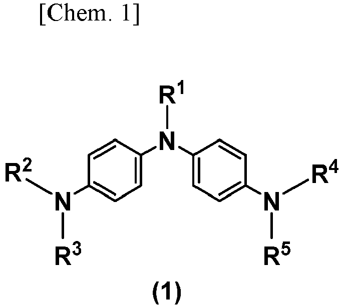 wherein R¹ to R⁵ are each independently a hydrogen atom or a group of formula (2): 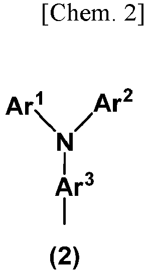 (wherein Ar¹ and Ar² are each independently an aryl group of 6 to 20 carbon atoms, Ar³ is an arylene group of 6 to 20 carbon atoms, and any two of Ar¹ to Ar³ may be bonded to form a ring with a nitrogen atom)
and at least one of R¹ to R⁵ is a hydrogen atom;
with a carbazole compound of formula (3): 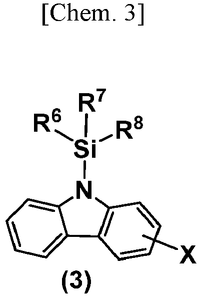 wherein X is a halogen atom or a pseudohalogen group, R⁶ to R⁸ are alkyl groups of 1 to 20 carbon atoms or aryl groups of 6 to 20 carbon atoms which may be substituted with Z¹, and Z¹ is an alkoxy group of 1 to 20 carbon atoms, a halogen atom, a nitro group, or a cyano group,
in the presence of a catalyst and a base, followed by deprotecting the silyl group.

2. The method for producing an aniline derivative of claim 1, wherein R⁶ to R⁸ are alkyl groups of 1 to 10 carbon atoms or aryl groups of 6 to 10 carbon atoms.

3. The method for producing an aniline derivative of claim 2, wherein two of R⁶ to R⁸ are methyl groups and the remaining one is a t-butyl group.

4. The method for producing an aniline derivative of any one of claims 1 to 3, wherein the base is t-butoxy sodium.

5. The method for producing an aniline derivative of any one of claims 1 to 4, wherein the catalyst is a palladium catalyst.

6. The method for producing an aniline derivative of any one of claims 1 to 5, wherein the coupling reaction is performed at 40 to 100°C.

7. The method for producing an aniline derivative of any one of claims 1 to 6, wherein the deprotection is performed using a fluoride ion.

8. The method for producing an aniline derivative of any one of claims 1 to 7, wherein R¹ to R⁵ are all hydrogen atoms.
